# EUROPÄISCHE PATENTSCHRIFT

(11) **EP 3 909 616 B1**
(45) Veröffentlichungstag und Bekanntmachung des Hinweises auf die Patenterteilung: **08.04.2026**
(21) Anmeldenummer: 20174268.1
(22) Anmeldetag: 12.05.2020
(51) Int. Cl.: A61L 2/10, A61L 9/20, A61G 3/00

(54) **RETTUNGSWAGEN MIT UV-DESINFEKTIONSVORRICHTUNG**
AMBULANCE WITH UV DISINFECTION DEVICE
AMBULANCE AVEC DISPOSITIF DE DÉSINFECTION UV

(43) Veröffentlichungstag der Anmeldung: 17.11.2021
(73) Patentinhaber: Fraunhofer-Gesellschaft zur Förderung der angewandten Forschung e.V., 80686 München (DE)
(72) Erfinder: RITTINGHAUS, Ernst-Wilhelm, 98693 Ilmenau (DE); WESTERHOFF, Thomas, 98693 Ilmenau (DE)
(74) Vertreter: Maiwald GmbH

(56) Entgegenhaltungen:
- WO-A1-2014/088298
- WO-A1-2019/117853
- CN-A- 108 962 882
- CN-A- 109 330 777
- CN-U- 202 044 428
- CN-U- 208 615 792
- CN-U- 209 316 267
- US-A1- 2007 053 188
- US-A1- 2019 091 738
- US-B1- 10 302 318
- US-B1- 9 855 353
- DATABASE WPI Week 201968, Derwent World Patents Index; AN 2019-75812E

## Beschreibung

Die vorliegende Erfindung betrifft eine Desinfektionsvorrichtung, insbesondere zur Desinfektion von Oberflächen und Luft, vorzugsweise in einer Sanitätseinrichtung, wie beispielsweise einem Rettungswagen. Weitere Aspekte der Erfindung betreffen eine Sanitätseinrichtung mit einer solchen Desinfektionsvorrichtung, eine Verwendung einer solchen Desinfektionsvorrichtung zur Desinfektion von Oberflächen und/oder Luft sowie ein Verfahren zur Desinfektion von Oberflächen und/oder Luft mit einer solchen Desinfektionsvorrichtung.

Die Desinfektionsvorrichtung umfasst eine UV-Strahlungseinrichtung zur Emission von UV-Strahlung, insbesondere zur Emission von UV-C-Strahlung, zur Desinfektion insbesondere von Oberflächen und/oder Luft. Unter dem Begriff "Desinfektion" wird vorliegend die Inaktivierung von Bakterien, Viren und Schimmelpilzen verstanden. Dies kann durch UV-Strahlung geschehen, da UV-Strahlung, insbesondere UV-C-Strahlung, chemische Bindungen in den Nukleinsäuren der DNA abhängig von Wellenlänge und einwirkender Dosis zerstört.

Im Stand der Technik sind Desinfektionsvorrichtungen bekannt, die als UV-Strahlungseinrichtung Quecksilberdampflampen einsetzen. Solche Quecksilberdampflampen sind jedoch einerseits relativ platzintensiv und umständlich zu handhaben,. Andererseits besitzen Quecksilberdampflampen keine Langzeitstabilität.

CN109 330 777 A offenbart einen Rettungsewagen mit UV-Sterilisator im Deckenbereich zur Bestrahlung des Innenraums des Rettungsewagens.

Daher ist die Aufgabe der vorliegenden Erfindung, eine Desinfektionsvorrichtung bereitzustellen, die besonders einfach, platzsparend, effektiv und langzeitstabil ist.

Diese Aufgabe wird dadurch gelöst, dass die UV-Strahlungseinrichtung zur Emission der UV-Strahlung mindestens eine UV-Leuchtdiode umfasst, d.h. eine Leuchtdiode oder sogenannte "LED", die zur Emission von UV-Strahlung angepasst ist. Solche UV-Leuchtdioden sind besonders klein, einfach und flexibel einsetzbar, können auch bei Wellenlängen oberhalb von 254 nm betrieben werden, wobei sich herausgestellt hat, dass bei Wellenlängen oberhalb von 254 nm eine erhöhte Desinfektionswirkung erzielt wird, und sind außerdem noch besonders langzeitstabil. Weitere Vorteile der UV-Leuchtdioden sind eine hohe mechanische Stabilität, das Wegfallen einer Aufheizphase, die Möglichkeit einer variablen geometrischen Anordnung im Array, die Möglichkeit einer Niederspannungsversorgung und eine extrem hohe Strahlungsdichte.

In einer bevorzugten Ausführungsform ist die mindestens eine UV-Leuchtdiode angepasst, um zur Desinfektion mit einer Hauptemission bei einer Wellenlänge von zwischen 260 nm und 280 nm, vorzugsweise zwischen 260 nm und 275 nm, weiter bevorzugt zwischen 260 nm und 270 nm, noch weiter bevorzugt zwischen 263 nm und 267 nm, meist bevorzugt 265 nm, betrieben zu werden, wobei vorzugsweise +/- 5 nm Toleranz zugelassen werden. Bei Wellenlängen um 265 nm wird Untersuchungen zufolge die höchste Desinfektionswirkung im nutzbaren Spektrum erreicht, die 50% über der Desinfektionswirkung bei 240 nm liegen kann. Unterhalb von 240 nm steigt die Desinfektionswirkung zwar auch stark an, dieser Bereich kann jedoch nicht genutzt werden, da in diesem Spektrum Ozon ausgebildet wird, das nicht nur schädlich ist, sondern auch der Wirkung der UV-C-Strahlung entgegensteht. In diesem Zusammenhang ist die Wellenlänge der Hauptemission diejenige, bei der die höchste Intensität gegeben ist.

In einer weiteren bevorzugten Ausführungsform ist die mindestens eine UV-Leuchtdiode angepasst, um zur Desinfektion eine Ausgangsleistung von zwischen 50 mW und 200 mW, vorzugsweise zwischen 80 mW und 150 mW, weiter bevorzugt mindestens 100 mW, meist bevorzugt 100 mW zu erzeugen. Bei einer solchen Ausgangsleistung der UV-Leuchtdioden kann mit einer überschaubaren Anzahl an UV-Leuchtdioden in einem überschaubaren Zeitrahmen eine sehr wirksame Desinfektion erreicht werden. Beispielsweise können UV-C-Leuchtdioden der Firma Bolb Inc., CA 94551, USA verwendet werden, beispielsweise solche vom Typ S6060-W268-P100-KL.

In einer weiteren bevorzugten Ausführungsform ist die UV-Strahlungseinrichtung angepasst, um zur Desinfektion bei einer Bestrahlungsdauer von 10 min vorzugsweise mit einem Energieeintrag von zwischen 150 J/m² und 400 J/m², weiter bevorzugt zwischen 200 J/m² und 350 J/m², meist bevorzugt zwischen 250 J/m² und 300 J/m², eine Desinfektionsleistung von mindestens vier log-Stufen, d.h. 99,99%, zu erreichen und/oder bei einer Bestrahlungsdauer von 20 min vorzugsweise mit einem Energieeintrag von zwischen 300 J/m² und 800 J/m², weiter bevorzugt zwischen 400 J/m² und 700 J/m², meist bevorzugt zwischen 500 J/m² und 600 J/m², eine Desinfektionsleistung von mindestens fünf log-Stufen, d.h. 99,999%, zu erreichen. Eine Desinfektionsleistung von vier log-Stufen, d.h. 99,99%, ist zur Inaktivierung der am häufigsten auftretenden Bakterien und Viren ausreichend, und bei einer Desinfektionsleistung von fünf log-Stufen, d.h. 99,999%, können auch Viren mit sehr hoher Infektionsrate inaktiviert werden. Eine Bestrahlungsdauer von 10 min ist beispielsweise geeignet, um den Sanitätsraum in einem Rettungswagen auf der kurzen Fahrt zu einem Einsatz zu 99,99% zu desinfizieren. Da die Wellenlängen der UV-Leuchtdioden eine höhere Effizienz bei der Schädigung der DNA als beispielsweise Quecksilberdampflampen aufweisen, kann diese Desinfektionsleistung mit einer Quecksilberdampflampe erst nach deutlich längerer Bestrahlungsdauer bzw. deutlich höherer Dosis erreicht werden, was einem kurzfristigen Einsatz im Rettungswagen entgegensteht.

In einer weiteren bevorzugten Ausführungsform umfasst die UV-Strahlungseinrichtung zwischen acht und 480 UV-Leuchtdioden, vorzugsweise zwischen 48 und 320 Leuchtdioden, meist bevorzugt 144 UV-Leuchtdioden, zur Emission der UV-Strahlung. Eine solche Anzahl an UV-Leuchtdioden kann die erforderliche Desinfektionsleistung bereitstellen und gleichzeitig in platzsparender Weise in die Desinfektionsvorrichtung integriert werden.

In einer weiteren bevorzugten Ausführungsform ist die UV-Strahlungseinrichtung modular aufgebaut. Der modulare Aufbau umfasst eine vorbestimmte Anzahl an Hauptplatinen, vorzugsweise zwei bis 12, weiter bevorzugt sechs bis 10, meist bevorzugt acht Hauptplatinen. Jeder Hauptplatine ist eine vorbestimmte Anzahl an UV-Strahlungsmodulen zugeordnet, vorzugsweise zwei bis vier, meist bevorzugt drei UV-Strahlungsmodule, die von der jeweiligen Hauptplatine versorgt und gesteuert werden. Jedes UV-Strahlungsmodul weist eine vorbestimmte Anzahl an nebeneinander angeordneten UV-Leuchtdioden auf, vorzugsweise zwei bis 10, weiter bevorzugt vier bis acht, meist bevorzugt sechs UV-Leuchtdioden. Ein solcher modularer Aufbau kann sehr flexibel erweitert oder reduziert werden. Die genannte Anzahl an Hauptplatinen, UV-Strahlungsmodulen und UV-Leuchtdioden kann die erforderliche Desinfektionsleistung bereitstellen und gleichzeitig in platzsparender und effektiver Weise in die Desinfektionsvorrichtung integriert werden.

Alle Platinen werden vorzugsweise mittels CAN-Bus gesteuert und verfügen über eine separate Energieeinspeisung. Hierdurch können mehrere Platinen modular zusammengeschaltet werden. Die Adressierung erfolgt vorzugsweise mittels DIP-Schalter und verfügt über 128 mögliche Adressen, was eine hohe Flexibilität garantiert.

In einer weiteren bevorzugten Ausführungsform weist die Desinfektionsvorrichtung eine Trägerstruktur auf, auf der die UV-Strahlungseinrichtung befestigt ist. Die Trägerstruktur ist vorzugsweise als Kühlkörper zur Aufnahme bzw. Ableitung der Verlustleistung der Hauptplatinen und der UV-Leuchtdioden ausgebildet. Auf diese Weise kann bei begrenzter Einsatzdauer auf eine aktive Luftkühlung verzichtet werden. An der Trägerstruktur können außerdem beispielsweise Griffe zum Halten oder Ausrichten der Desinfektionsvorrichtung sowie ein Bedienungspanel zur manuellen Eingabe von Steuerbefehlen angeordnet sein.

Dabei ist es besonders bevorzugt, wenn die Trägerstruktur mindestens einen länglichen Profilträger, vorzugsweise aus Aluminium, insbesondere Aluminium-Strangguss, aufweist, auf dem die Hauptplatinen und die zugeordneten UV-Leuchtdioden vorzugsweise linear hintereinander angeordnet sind. Die Hauptplatinen und/oder die UV-Leuchtdioden können jedoch auch in anderer Form, beispielsweise in kreisförmiger, runder oder abgewinkelter Folge, auf dem Profilträger angeordnet sein. Solche Aluminium-Strangguss-Profilträger bilden effektive Kühlkörper. Der mindestens eine Profilträger weist vorzugsweise eine Vielzahl von Kühlrippen zur Ableitung der Verlustleistung auf, die sich im Bereich der Hauptplatinen und der UV-Leuchtdioden auf der Rückseite des Profilträgers von Hauptplatine und UV-Leuchtdiode wegerstrecken. Die UV-Leuchtdioden sind vorzugsweise mit Abdeckungen aus Quarzglas-Ronden bedeckt und die Hauptplatinen sind vorzugsweise mit Abdeckungen aus Kunststoff bedeckt. Quarzglas-Ronden absorbieren die UV-C-Strahlung nicht oder nur unwesentlich. Die Abdeckung der Hauptplatinen ist vorzugsweise aus einem nicht vollständig transparenten Kunststoffglas gebildet, um eine diffuse Lichtverteilung von auf den Hauptplatinen vorgesehenen RGB- und Weißlicht-Leuchtdioden zu erzeugen.

Dabei ist es ferner bevorzugt, wenn die Trägerstruktur eine Basisplatte und zwei Profilträger aufweist, die an gegenüberliegenden Enden der Basisplatte parallel zueinander, insbesondere in symmetrischer Weise, auf der Basisplatte angebracht sind. Vorzugsweise sind auf jedem Profilträger in einer Reihe nacheinander vier Hauptplatinen mit jeweils drei zugeordneten UV-Strahlungsmodulen mit jeweils sechs UV-Leuchtdioden angeordnet, die vorzugsweise parallel versetzt zu den Hauptplatinen angeordnet sind. Dies stellt eine besonders kompakte und effektive Bauweise der Desinfektionsvorrichtung dar.

In einer bevorzugten Ausführungsform weist die Desinfektionsvorrichtung eine oder mehrere weitere Leuchtdioden zur Emission von Weißlicht und/oder RGB-Licht auf, die vorzugsweise direkt auf einer Hauptplatine angeordnet sind. Vorzugsweise weist jede Hauptplatine mindestens eine Weißlicht-Leuchtdiode und mindestens eine RGB-Leuchtdiode auf. Die Weißlicht- bzw. RGB-Leuchtdioden können zur Beleuchtung verwendet werden, beispielsweise zur Beleuchtung eines Sanitätsraums, in dem die Desinfektionsvorrichtung verwendet wird.

Ein weiterer Aspekt der Erfindung betrifft eine Sanitätseinrichtung umfassend eine Desinfektionsvorrichtung nach einer der zuvor beschriebenen Ausführungsformen. Die Sanitätseinrichtung kann eine mobile Sanitätseinrichtung oder eine stationäre Sanitätseinrichtung sein und insbesondere einen Sanitätsraum aufweisen. Die zuvor im Zusammenhang mit der Desinfektionsvorrichtung beschriebenen Merkmale und Wirkungen sind vis-a-vis auf die Sanitätseinrichtung anwendbar und bevorzugt.

In einer bevorzugten Ausführungsform ist die Sanitätseinrichtung ein Rettungswagen. Die Desinfektionsvorrichtung ist vorzugsweise in einem Sanitätsraum im Inneren des Rettungswagens vorgesehen, so dass bei Betätigung der Desinfektionsvorrichtung sowohl die Luft als auch die Oberflächen in dem Rettungswagen bzw. Sanitätsraum desinfiziert werden. Die Desinfektionsvorrichtung kann in einem Rettungswagen sehr effektiv eingesetzt werden, da der Rettungswagen damit in sehr kurzer Zeit, beispielsweise bei der Fahrt zu einem Einsatzort, desinfiziert werden kann. Ferner können auch gezielt einzelne zur Behandlung eines Patienten erforderliche Instrumente desinfiziert werden. Alternativ zu einem Rettungswagen kann die Sanitätseinrichtung jedoch beispielsweise auch als stationärer Sanitätsraum, beispielsweise in einem Krankenhaus oder einer Arztpraxis, oder als mobiler Sanitätsraum in Krisengebieten ausgebildet sein.

Dabei ist es besonders bevorzugt, wenn die Desinfektionsvorrichtung an einem Deckencenter oder Deckenpanel des Rettungswagens angeordnet ist. Das Deckencenter ist an der Decke des im Inneren des Rettungswagens vorgesehenen Sanitätsraums angeordnet und ist ein besonders geeigneter Ort für die Desinfektionsvorrichtung, da sie dort wenig Platz in Anspruch nimmt und die emittierte UV-Strahlung den gesamten Sanitätsraum, insbesondere sämtliche Luft und Oberflächen in dem Sanitätsraum des Rettungswagens, erreichen kann. Gleichzeitig kann sie dort mittels RBG- bzw. Weißlich-Leuchtdioden auch die Deckenbeleuchtung des Sanitätsraums übernehmen.

Ein weiterer Aspekt der vorliegenden Erfindung betrifft eine Verwendung einer Desinfektionsvorrichtung nach einer der zuvor beschriebenen Ausführungsformen zur Desinfektion von Oberflächen und/oder Luft, vorzugsweise in Sanitätseinrichtungen. Die zuvor im Zusammenhang mit der Desinfektionsvorrichtung beschriebenen Merkmale und Wirkungen sind vis-a-vis auf die vorliegende Verwendung dieser anwendbar und bevorzugt.

Ein weiterer Aspekt der vorliegenden Erfindung betrifft ein Verfahren zur Desinfektion von Oberflächen und/oder Luft, vorzugsweise in Sanitätseinrichtungen, mit den Schritten: a) Bereitstellen einer Desinfektionsvorrichtung nach einer der zuvor beschriebenen Ausführungsformen und b) Betreiben der Desinfektionsvorrichtung derart, dass die UV-Strahlungseinrichtung UV-Strahlung in Richtung der zu desinfizierenden Oberflächen und/oder Luft emittiert. Die zuvor im Zusammenhang mit der Desinfektionsvorrichtung beschriebenen Merkmale und Wirkungen sind vis-a-vis auf das vorliegende Verfahren anwendbar und bevorzugt.

Ein bevorzugtes Ausführungsbeispiel der vorliegenden Erfindung wird im Folgenden anhand einer Zeichnung näher erläutert. Die Zeichnung zeigt in
- Fig. 1: eine perspektivische Ansicht einer erfindungsgemäßen Desinfektionsvorrichtung,
- Fig. 2: eine perspektivische Ansicht eines isolierten Profilträgers der Desinfektionsvorrichtung aus Fig. 1,
- Fig. 3: eine Draufsicht auf den Profilträger aus Fig. 2,
- Fig. 4: eine schematische Darstellung eines Rettungswagens mit einer Detailansicht eines darin vorgesehenen Sanitätsraums, in der eine Desinfektionsvorrichtung gemäß Fig.1 vorgesehen ist, und
- Fig. 5: eine Simulation der Desinfektionsleistung durch Bestrahlung mit der Desinfektionsvorrichtung in dem Sanitätsraum des Rettungswagens aus Fig. 4.

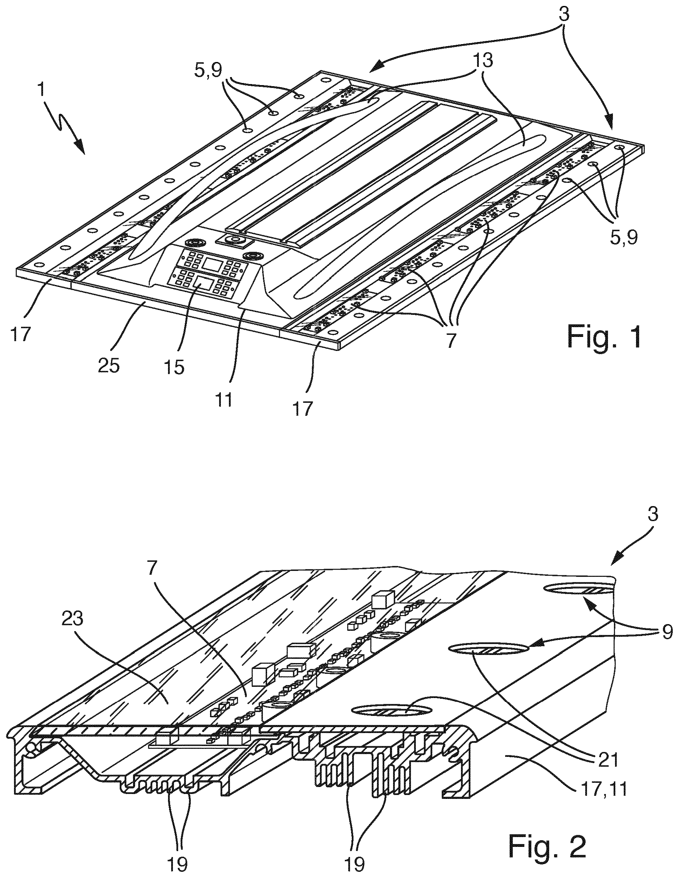

In Fig. 1 ist eine erfindungsgemäße Desinfektionsvorrichtung 1 dargestellt. Die Desinfektionsvorrichtung 1 umfasst eine UV-Strahlungseinrichtung 3 zur Emission von UV-C-Strahlung zur Desinfektion von Oberflächen und/oder Luft. Die UV-Strahlungseinrichtung 3 umfasst zur Emission der UV-C-Strahlung 144 UV-Leuchtdioden 5 mit einer Ausgangsleistung von 100 mW, die angepasst sind, um zur Desinfektion mit einer Hauptemission bei einer Wellenlänge von 265 nm +/- 5 nm Toleranz betrieben zu werden.

Damit ist die UV-Strahlungseinrichtung 3 angepasst, um bei einer Bestrahlungsdauer von 10 min mit einem Energieeintrag von zwischen 250 J/m² und 300 J/m² eine Desinfektionsleistung von in etwa vier log-Stufen, d.h. 99,99%, zu erreichen, und bei einer Bestrahlungsdauer von 20 min mit einem Energieeintrag von zwischen 500 J/m² und 600 J/m² eine Desinfektionsleistung von in etwa fünf log-Stufen, d.h. 99,999%, zu erreichen.

Die UV-Strahlungseinrichtung 3 ist modular aufgebaut umfassend acht Hauptplatinen 7, wobei jeder Hauptplatine 7 drei UV-Strahlungsmodule 9 zugeordnet sind, die von der jeweiligen Hauptplatine 7 versorgt und gesteuert werden, und wobei jedes UV-Strahlungsmodul 9 sechs nebeneinander angeordnete UV-Leuchtdioden 5 aufweist. Ferner weist jede Hauptplatine 7 eine Weißlicht-Leuchtdiode und eine RGB-Leuchtdiode auf, die zur Beleuchtung verwendet werden können.

Die Desinfektionsvorrichtung 1 weist eine Trägerstruktur 11 auf, auf der die UV-Strahlungseinrichtung 3 befestigt ist. Die Trägerstruktur 11 ist als Kühlkörper zur Aufnahme bzw. Ableitung der Verlustleistung der Hauptplatinen 7 und der UV-Leuchtdioden 5 ausgebildet. An der Trägerstruktur 11 sind außerdem Griffe 13 zum Halten oder Ausrichten der Desinfektionsvorrichtung 1 sowie ein Bedienungspanel 15 zur manuellen Eingabe von Steuerbefehlen angeordnet.

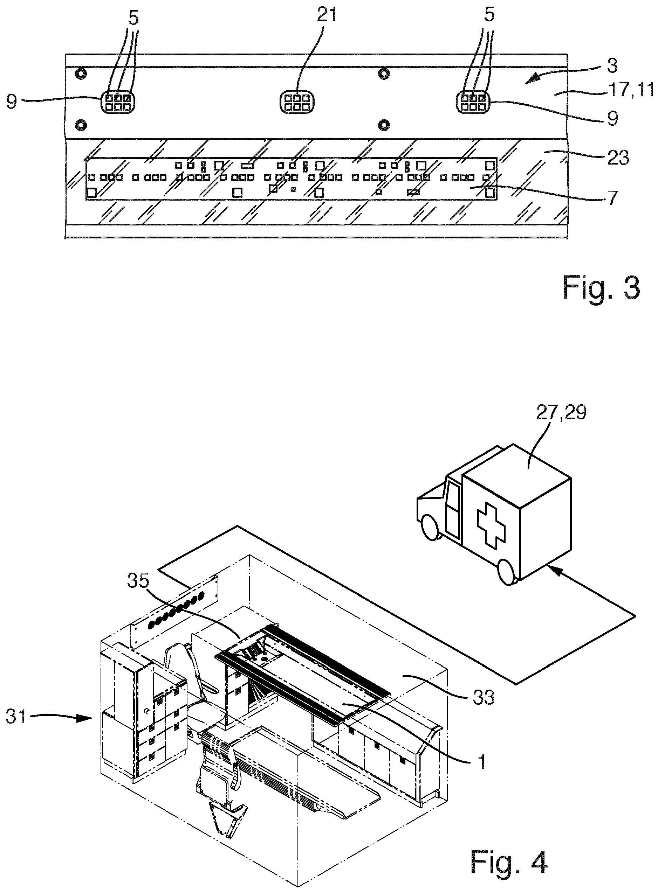

Die Trägerstruktur 11 weist zwei längliche Profilträger 17 aus Aluminium-Strangguss auf, auf denen die Hauptplatinen 7 und die zugeordneten UV-Leuchtdioden 5 linear hintereinander angeordnet sind und von denen einer in den Fign. 2 und 3 näher dargestellt ist. Wie in Fig. 2 gezeigt, weisen die Profilträger 17 eine Vielzahl von Kühlrippen 19 zur Ableitung der Verlustleistung auf, die sich im Bereich der Hauptplatinen 7 und der UV-Leuchtdioden 5 auf der Rückseite des Profilträgers 17 von Hauptplatine 7 und UV-Leuchtdiode 5 wegerstrecken. Wie ebenfalls in Fig. 2 gezeigt, sind die UV-Leuchtdioden 5 mit transparenten Abdeckungen 21 aus Quarzglas-Ronden bedeckt, während die Hauptplatinen 7 mit nicht vollständig transparenten Abdeckungen 23 aus Kunststoffglas bedeckt sind, die eine diffuse Lichtverteilung von den auf den Hauptplatinen 7 vorgesehenen RGB- und Weißlicht-Leuchtdioden zu erzeugen.

Wie in Fig. 1 erkennbar, sind die zwei Profilträger 17 an gegenüberliegenden Enden einer Basisplatte 25 parallel und symmetrisch zueinander auf der Basisplatte 25 angebracht. Dabei sind auf jedem Profilträger 17 in einer Reihe nacheinander vier Hauptplatinen 7 mit jeweils drei zugeordneten UV-Strahlungsmodulen 9 mit jeweils sechs UV-Leuchtdioden 5 angeordnet, die parallel versetzt zu den Hauptplatinen 7 angeordnet sind.

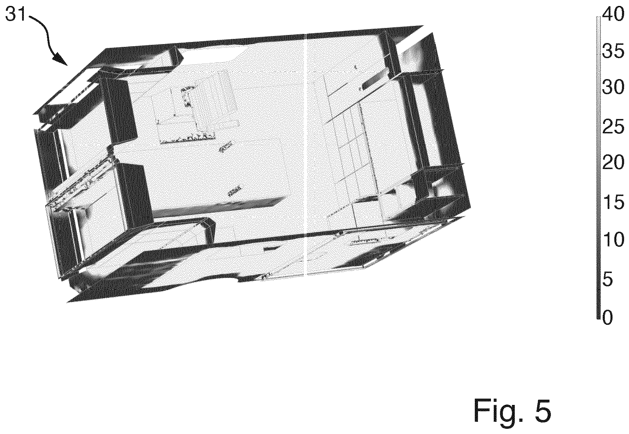

Die Desinfektionsvorrichtung 1 kann zur Desinfektion in Sanitätseinrichtungen 27, wie beispielsweise einem Rettungswagen 29, eingesetzt werden. Eine solche Sanitätseinrichtung 27 in Form eines Rettungswagens 29 ist in Fig. 4 dargestellt. Wie in Fig. 4 erkennbar ist, weist der Rettungswagen 29 einen Sanitätsraum 31 zum Transport und zur Notversorgung eines Patienten auf. Der Sanitätsraum 31 hat eine Decke 33, in der zentral ein Deckencenter 35 mit verschiedenen Funktionselementen vorgesehen ist. In dem in Fig. 4 gezeigten Ausführungsbeispiel ist in dem Deckencenter 35 auch die Desinfektionsvorrichtung 1 integriert, so dass bei Betätigung der Desinfektionsvorrichtung 1 sowohl die Luft als auch die Oberflächen in nahezu allen Bereichen des Sanitätsraums 31 des Rettungswagens 29 effektiv desinfiziert werden können. Dies wird in der in Fig. 5 dargestellten Simulation bestätigt, welche in dem Sanitätsraum 31 aus Fig. 4 den Energieeintrag nach 10 min bei einer Bestrahlung mit der in Fig. 1 gezeigten Desinfektionsvorrichtung 1, d.h. mit 144 UV-Leuchtdioden 5 mit je 100 mW Leistung bei 265 nm Wellenlänge darstellt, wobei die weißen Bereiche der Darstellung eine ausreichende Desinfektionsleistung mit 400 J/m² darstellen. Die Desinfektionsvorrichtung 1 kann in einem Rettungswagen sehr effektiv eingesetzt werden, da der Rettungswagen damit in sehr kurzer Zeit, beispielsweise bei der Fahrt zu einem Einsatzort, desinfiziert werden kann.

Die zuvor beschriebene Desinfektionsvorrichtung 1 ist besonders vorteilhaft, da die verwendeten UV-Leuchtdioden 5 besonders klein, einfach und flexibel einsetzbar sind, auch bei Wellenlängen oberhalb von 254 nm betrieben werden können und somit eine erhöhte Desinfektionswirkung haben und außerdem noch besonders langzeitstabil sind. Des Weiteren weisen die UV-Leuchtdioden 5 eine hohe mechanische Stabilität auf, benötigen keine Aufheizphase, können eine variable geometrische Anordnung im Array aufweisen und können eine Niederspannungsversorgung und eine extrem hohe Strahlungsdichte aufweisen.

## Patentansprüche

1. Rettungswagen (29) mit einem Deckencenter (35) oder mit einem Deckenpanel, wobei das Deckencenter oder das Deckenpanel mit einer Desinfektionsvorrichtung (1) ausgestattet sind
**dadurch gekennzeichnet, dass**
die Desinfektionsvorrichtung eine UV-Strahlungseinrichtung (3) mit 8 bis 480 UV-Leuchtdioden (5) umfasst, und dass die UV-Strahlungseinrichtung (3) angepasst ist, um zur Desinfektion bei einer Bestrahlungsdauer von 10 min mit einem Energieeintrag von zwischen 150 J/m² und 400 J/m² eine Desinfektionsleistung von mindestens vier log-Stufen zu erreichen.

2. Rettungswagen (29) gemäß Anspruch 1, wobei die Desinfektionseinrichtung (1) so betrieben wird, dass die UV-Strahlungseinrichtung (3) in Richtung der zu desinfizierenden Oberfläche und/oder Luft emittiert.

3. Rettungswagen (29) gemäß Anspruch 1 oder Anspruch 2, wobei die UV-Leuchtdioden (5) so angepasst sind, um zur Desinfektion mit einer Hauptemission von 260 bis 270 nm betrieben zu werden.

4. Rettungswagen (29) gemäß einem der voranstehenden Ansprüche, wobei die Desinfektionsvorrichtung (1) eine oder mehrere weitere Leuchtdioden zur Emission von Weißlicht und/oder RGB-Licht aufweist.

5. Rettungswagen (29) gemäß einem der voranstehenden Ansprüche, wobei die UV-Strahlungseinrichtung (3) so angepasst ist, dass bei einer Bestrahlungsdauer von 20 min mit einem Energieeintrag von zwischen 300 J/m2 und 800 J/m2 eine Desinfektionsleistung von mindestens 99,999% erreicht wird.

## Claims

1. Ambulance (29) with a ceiling center (35) or with a ceiling panel, wherein the ceiling center or the ceiling panel is equipped with a disinfection device (1),
**characterized in that**
the disinfection device comprises a UV radiation device (3) with 8 to 480 UV light-emitting diodes (5), and that the UV radiation device (3) is adapted to achieve a disinfection performance of at least four log levels for disinfection with an irradiation duration of 10 min and an energy input of between 150 J/m² and 400 J/m².

2. Ambulance (29) according to claim 1, wherein the disinfection device (1) is operated such that the UV radiation device (3) emits in the direction of the surface to be disinfected and/or the air.

3. Ambulance (29) according to claim 1 or claim 2, wherein the UV light-emitting diodes (5) are adapted to operate for disinfection with a primary emission of 260 to 270 nm.

4. Ambulance (29) according to any one of the preceding claims, wherein the disinfection device (1) comprises one or more additional light-emitting diodes for emitting white light and/or RGB light.

5. Ambulance (29) according to any one of the preceding claims, wherein the UV radiation device (3) is adapted such that a disinfection efficiency of at least 99.999% is achieved with an irradiation duration of 20 min and an energy input of between 300 J/m² and 800 J/m².

## Revendications

1. Ambulance (29) comprenant un centre de plafond (35) ou comprenant un panneau de plafond, dans laquelle le centre de plafond ou le panneau de plafond est équipé d'un dispositif de désinfection (1)
**caractérisé en ce que**
le dispositif de désinfection comprend une installation à rayonnement UV (3) comprenant 8 à 480 diodes luminescentes UV (5), et que l'installation à rayonnement UV (3) est adaptée pour atteindre pour la désinfection une efficacité de désinfection d'au moins quatre niveaux log à une durée d'irradiation de 10 min avec un apport d'énergie compris entre 150 J/m² et 400 J/m².

2. Ambulance (29) selon la revendication 1, dans laquelle l'installation de désinfection (1) fonctionne de telle manière que l'installation à rayonnement UV (3) émette en direction de la surface et/ou de l'air à désinfecter.

3. Ambulance (29) selon la revendication 1 ou la revendication 2, dans laquelle les diodes luminescentes UV (5) sont adaptées pour fonctionner pour la désinfection avec une émission principale de 260 à 270 nm.

4. Ambulance (29) selon l'une des revendications précédentes, dans laquelle le dispositif de désinfection (1) présente une ou plusieurs autres diodes luminescentes pour l'émission de lumière blanche et/ou de lumière RVB.

5. Ambulance (29) selon l'une des revendications précédentes, dans laquelle le l'installation à rayonnement UV (3) est adaptée de telle sorte qu'à une durée d'irradiation de 20 min, avec un apport d'énergie compris entre 300 J/m² et 800 J/m², une efficacité de désinfection d'au moins 99,999 % soit atteinte.
